(19) Europäisches Patentamt
European Patent Office
Office européen des brevets

(11) **EP 3 791 781 A1**

(12) **EUROPEAN PATENT APPLICATION**
published in accordance with Art. 153(4) EPC

(43) Date of publication:
**17.03.2021 Bulletin 2021/11**

(51) Int Cl.:
**A61B 5/022** (2006.01)    **A61B 5/0225** (2006.01)
**A61B 5/153** (2006.01)    **A61B 17/132** (2006.01)

(21) Application number: **19800040.8**

(22) Date of filing: **10.05.2019**

(86) International application number:
**PCT/JP2019/018813**

(87) International publication number:
**WO 2019/216432 (14.11.2019 Gazette 2019/46)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **10.05.2018 JP 2018091056**

(71) Applicant: **Techno Science Co., Ltd.
Numazu-shi, Shizoka 410-0314 (JP)**

(72) Inventors:
• **TAKANO, Jun
Numazu-shi, Shizuoka 410-0314 (JP)**
• **TAKADA, Makoto
Numazu-shi, Shizuoka 410-0314 (JP)**

(74) Representative: **Markfort, Iris-Anne Lucie
Lorenz & Kollegen
Patentanwälte Partnerschaftsgesellschaft mbB
Alte Ulmer Straße 2
89522 Heidenheim (DE)**

(54) **HEMOSTASIS AID AND TOURNIQUET**

(57)    [Object] To provide a hemostasis aid and a tourniquet by which a blood vessel at a puncture site can be more reliably distended. [Solving Means] A hemostasis aid 1 includes a tourniquet 10 including a plurality of pressurizing layers 11a to 11e divided in a width direction and a control device 20 that pressurizes the pressurizing layers 11a to 11e sequentially from the pressurizing layer 11a on one end side A in the width direction W to on the other end side B. Accordingly, a puncture site can be reliably distended by wrapping the tourniquet 10 such that the puncture site is positioned on a patient's distal side with respect to the other end side.

FIG.1

## Description

Technical Field

The present invention relates to a hemostasis aid and a tourniquet which are used during puncture for blood sampling, drip injection, or the like or during shunt puncture for a dialysis, for example.

Background Art

A tourniquet is used for expelling blood and distending a blood vessel during puncture. If the pressure of this tourniquet is too high, it may cause capillary bleeding, subcutaneous bleeding, and the like and it may be often painful. Therefore, it is favorable that the pressure of the tourniquet is not too high and the tourniquet time is as short as possible.

Patent Literature 1 discloses a simple auto electronic tourniquet which does not inflict pain on a person receiving the tourniquet in a preparatory stage before venipuncture and which shortens the time required in preparation for venipuncture. This simple auto electronic tourniquet makes it possible to detect a change in a biological signal and is configured to set a pressure of a manchette when the change in the biological signal is detected as a target pressure and to control the pressure of the manchette to fall within a range closer to the target pressure.

Citation List

Patent Literature

Patent Literature 1: Japanese Patent Application Laid-open No. 2013-118938

Disclosure of Invention

Technical Problem

It is necessary to consider the pressure of the tourniquet as described in Patent Literature 1 for efficiently expelling blood and distending a blood vessel at a puncture site.

However, even if the pressure of the tourniquet is sufficiently controlled, the blood vessel at the puncture site may be insufficiently distended.

In view of the above-mentioned circumstances, it is an object of the present invention to provide a hemostasis aid and a tourniquet by which a blood vessel at a puncture site can be more reliably distended.

Solution to Problem

In order to accomplish the above-mentioned object, a hemostasis aid according to an embodiment of the present invention includes a tourniquet and a control device that controls a pressure of the tourniquet, which is configured such that a region pressurized by the tourniquet extends or moves from one end side to another end side in a width direction of the tourniquet.

This tourniquet is wrapped around a patient's arm such that on the one end side in the width direction of the tourniquet is positioned on the patient's proximal side (side closer to heart) that is an attached site during tourniquet application. In veins, blood flows from the patient's distal side (side closer to hand) to the patient's proximal side. Therefore, when the region pressurized by the tourniquet is configured to extend or move from the one end side in the width direction of the tourniquet (heart side) to the other end side (hand side), the vein portion compressed by the tourniquet extends or moves in a direction opposite to the direction in which the blood flows. Accordingly, blood flowing into this region is compressed in the vein, flows backward (flows from the heart side to the hand side), and joins blood flowing from the patient's distal side (side closer to hand), and the vein blood vessel is greatly distended. Therefore, in accordance with the present invention, the blood vessel at the puncture site can be more reliably distended.

In the hemostasis aid according to the embodiment of the present invention, the tourniquet may include a plurality of pressurizing layers divided in the width direction, and the control device may perform pressurizing sequentially from a pressurizing layer on the one end side in the width direction to a pressurizing layer on the other end side.

Accordingly, the extension or movement of the region pressurized by the tourniquet from the one end side to the other end side in the width direction of the tourniquet can be correctly performed, and the blood vessel at the puncture site can be more reliably distended.

In the hemostasis aid according to the embodiment of the present invention, at least one of the plurality of pressurizing layers may include a cutout portion at a corner on the other end side in a pressurizing surface of the pressurizing layer.

When the vein is pressed by the pressurizing layer including such a cutout portion, the blood in the pressed region is more likely to be pushed to the patient's distal side (side closer to hand) due to this press. That is, the backward flow rate of blood in the pressed region increases. It contributes to the distention of the blood vessel at the puncture site.

In the hemostasis aid according to the embodiment of the present invention, the control device may perform pressurizing sequentially from the pressurizing layer on the one end side to the pressurizing layer on the other end side again in accordance with an operation of additional tourniquet application.

Accordingly, since similar pressurizing can be continuously performed by the tourniquet, the blood vessel at the puncture site can be more reliably distended without taking much time.

In the hemostasis aid according to the embodiment of the present invention, the control device may pressurize, when the operation of additional tourniquet application is performed, the pressurizing layer on the other end side, which is pressurized before the re-pressurizing, until at least the pressurizing layer on the one end side is re-pressurized.

Accordingly, the distension before re-pressurizing can be maintained and re-pressurizing can be performed under that state, the blood vessel at the puncture site can be more reliably distended.

In the hemostasis aid according to the embodiment of the present invention, the control device may pressurize, when the operation of additional tourniquet application is performed, the pressurizing layer on the other end side, which is pressurized before the re-pressurizing, to maintain a pressure higher than a pressure before the re-pressurizing.

Accordingly, it is possible to suitably maintain the inflation of the pressurizing layer on the other end side, and the blood can be prevented from flowing from the patient's hand side to the heart side in veins during additional tourniquet application.

In the hemostasis aid according to the embodiment of the present invention, the control device may measure a blood pressure via the tourniquet and control a pressure of the tourniquet to be a tourniquet pressure according to the measured blood pressure.

In the hemostasis aid according to the embodiment of the present invention, the control device may determine a systolic pressure and a diastolic pressure on the basis of the measured blood pressure, determines a blood pressure mean value in accordance with (systolic pressure + diastolic pressure)/2, and determine a tourniquet pressure in accordance with (blood pressure mean value + diastolic pressure)/2.

Accordingly, it is unnecessary to boost the pressure by applying unnecessary pressure, which imposes a burden on the patient, unlike traditional blood pressure measurement.

Here, the tourniquet may include a microphone for collecting Korotkov sounds. The control device may set a pressure measured when Korotkov sounds can be sensed via the microphone in a process of boosting the pressure of the pressurizing layer as a diastolic pressure and set a pressure measured when the Korotkov sounds disappear, which is sensed via the microphone, in a process of further boosting the pressure of the pressurizing layer as a systolic pressure.

Accordingly, it is possible to set the tourniquet pressure to an optimal tourniquet pressure without taking much time.

In the hemostasis aid according to the embodiment of the present invention, the control device may measure a pressure waveform via the tourniquet during tourniquet application, set a highest value of the measured pressure waveform as a systolic pressure, set a lowest value of the measured pressure waveform as a diastolic pressure, and control the pressure of the tourniquet to maintain the tourniquet pressure on the basis of the systolic pressure and the diastolic pressure.

Accordingly, it is possible to perform continuous blood pressure measurement without measuring a blood pressure multiple times and to cope with the patient's condition change in case of emergency or the like.

In the hemostasis aid according to the embodiment of the present invention, the control device may include a pressurizing pump that feeds a pressurizing fluid to each of the pressurizing layers, a branch path that branches the pressurizing fluid fed from the pressurizing pump into each of the pressurizing layers, a plurality of solenoid valves that opens and closes each of flow paths branched by the branch path, a plurality of exhaust valves for exhausting each of the flow paths branched and a control unit that controls an operation of the pressurizing pump, open/close of each of the solenoid valves, and open/close of each of the exhaust valves.

Accordingly, it is possible to perform pressurizing sequentially from the pressurizing layer on the one end side to the pressurizing layer on the other end side with a reduced number of pressurizing pumps. Therefore, cost saving and downsizing of the hemostasis aid according to the embodiment of the present invention can be achieved.

In the hemostasis aid according to the embodiment of the present invention, the control device may include a pressurizing pump that feeds the pressurizing fluid into each of the pressurizing layers, a multi-directional solenoid valve that branches the pressurizing fluid fed from the pressurizing pump into each of the pressurizing layers and opens and closes each of flow paths branched and a control unit that controls an operation of the pressurizing pump and open/close of the multi-directional solenoid valve.

Accordingly, it is possible to perform pressurizing sequentially from the pressurizing layer on the one end side to the pressurizing layer on the other end side with a reduced number of pressurizing pumps and valves. Therefore, cost saving and downsizing of the hemostasis aid according to the embodiment of the present invention can be achieved.

In the hemostasis aid according to the embodiment of the present invention, the control device may include a plurality of pressurizing and depressurizing pumps that is provided corresponding to the pressurizing layers, respectively, and that pressurizes and depressurizes each of the pressurizing layers and a control unit that controls an operation of each of the pressurizing and depressurizing pumps.

Accordingly, it is possible to perform pressurizing sequentially from the pressurizing layer on the one end side to the pressurizing layer on the other end side without using an exhaust mechanism and a branching mechanism.

In the hemostasis aid according to the embodiment of the present invention, the control device may include a pressurizing and depressurizing pump that pressurizes and depressurizes each of the pressurizing layers, a branch path that branches a pressurizing fluid fed from the pressurizing and depressurizing pump into each of the pressurizing layers, a plurality of solenoid valves that opens and closes each of flow paths branched by the branch path and a control unit that controls an operation of the pressurizing and depressurizing pump and open/close of each of the solenoid valves.

Accordingly, it is possible to perform pressurizing sequentially from the pressurizing layer on the one end side to the pressurizing layer on the other end side with a reduced number of pressurizing and depressurizing pumps without using an exhaust mechanism.

Here, since the pressurizing and depressurizing pumps are versatile components, the hemostasis aid according to the embodiment of the present invention can be realized at low cost by employing the pressurizing and depressurizing pumps. In addition, it is expected that the hemostasis aid has good accuracy and is less likely to malfunction.

In the hemostasis aid according to the embodiment of the present invention, the tourniquet may include a pressurizing member including a pressurizing surface and a cutout portion provided at a corner on the other end side in the pressurizing surface.

Accordingly, the extension or movement of the region pressurized by the tourniquet from the one end side to the other end side in the width direction of the tourniquet can be realized with a simple configuration.

A tourniquet according to an embodiment of the present invention includes a plurality of pressurizing layers divided in a width direction; and a band-like cover member that is capable of being wrapped around an attached site, in which the plurality of pressurizing layers is disposed to be adjacent to each other in the width direction inside the cover member.

In the tourniquet according to the embodiment of the present invention, the pressurizing layers may be respectively provided with fluid introduction outlets.

In the tourniquet according to the embodiment of the present invention, the fluid introduction outlets may have diameters gradually smaller sequentially from the pressurizing layer on one end side in the width direction to the pressurizing layer on another end side.

Accordingly, the region pressurized by the tourniquet is configured to expand from the one end side to the other end side in the width direction of the tourniquet with a simple configuration.

In the tourniquet according to the embodiment of the present invention, the pressurizing layers may each have capacity gradually larger sequentially from the pressurizing layer on one end side in the width direction to the pressurizing layer on another end side.

Accordingly, the region pressurized by the tourniquet is configured to expand from the one end side to the other end side in the width direction of the tourniquet with a simple configuration.

The tourniquet according to the embodiment of the present invention may further include flow paths that cause a fluid to flow between the adjacent pressurizing layers, in which the flow paths may have diameters gradually smaller from the one end side to the other end side in the width direction, and the pressurizing layer on the one end side may include a fluid introduction outlet having a diameter larger than the diameter of each of the flow paths. Accordingly, the region pressurized by the tourniquet is configured to expand from the one end side to the other end side in the width direction of the tourniquet with a simple configuration.

In the tourniquet according to the embodiment of the present invention, at least one of the plurality of pressurizing layers may include a cutout portion at a corner on one side of the width direction in the pressurizing surface of the pressurizing layer.

A tourniquet according to an embodiment of the present invention includes: a pressurizing member including a pressurizing surface and a cutout portion provided at a corner on one side in a width direction of the pressurizing surface; and a band-like cover member that accommodates the pressurizing member and is capable of being wrapped around an attached site. Advantageous Effects of Invention

In accordance with the present invention, a blood vessel at a puncture site can be more reliably distended.

Brief Description of Drawings

[Fig. 1] A block diagram showing a hemostasis aid according to an embodiment of the present invention.

[Fig. 2] A diagram showing a configuration of the tourniquet shown in Fig. 1.

[Fig. 3] A cross-sectional view taken along the line X-X of Fig. 2.

[Fig. 4A] A diagram for describing an action of a traditional tourniquet as a comparative example.

[Fig. 4B] A diagram for describing an action of pressurizing layers each including a cutout portion in the embodiment of the present invention.

[Fig. 5] A flowchart showing an operation on the hemostasis aid shown in Fig. 1.

[Fig. 6] A flowchart showing an operation of the hemostasis aid in connection with the operation shown in Fig. 5.

[Fig. 7] A graph showing a blood pressure waveform for describing a continuous blood pressure measurement method.

[Fig. 8] A timing chart showing a timing of pressurizing the pressurizing layers in a case where a person who performs puncture presses a start button and presses an end button without pressing an additional tourniquet application button in the hemostasis aid shown in Fig. 1.

[Fig. 9] A timing chart showing the timing of pressurizing the pressurizing layers in a case where the person who performs puncture presses the start button, presses the additional tourniquet application button, and presses the end button in the hemostasis aid shown in Fig. 1.

[Fig. 10] A diagram showing a configuration of a hemostasis aid according to another embodiment of the present invention.

[Fig. 11] A diagram showing a configuration of a hemostasis aid according to still another embodiment of the present invention.

[Fig. 12] A diagram showing a configuration of a hemostasis aid according to still another embodiment of the present invention.

[Fig. 13] A timing chart showing another example of the timing of pressurizing the pressurizing layers in a case where the person who performs puncture presses the start button and presses the end button without pressing the additional tourniquet application button in the hemostasis aid shown in Fig. 1.

[Fig. 14] A timing chart showing another example of the timing of pressurizing the pressurizing layers in a case where the person who performs puncture presses the start button, presses the additional tourniquet application button, and presses the end button in the hemostasis aid shown in Fig. 1.

[Fig. 15] A cross-sectional view showing a tourniquet according to a modified example (Part I) of the present invention.

[Fig. 16] A schematic plan view showing a tourniquet according to a modified example (Part II) of the present invention.

[Fig. 17] A schematic plan view showing a tourniquet according to a modified example (Part III) of the present invention.

[Fig. 18] A schematic plan view showing a tourniquet according to a modified example (Part IV) of the present invention.

[Fig. 19] A schematic cross-sectional view showing a tourniquet according to a modified example (Part V) of the present invention.

[Fig. 20] An explanatory diagram of a modified example of a pressurizing method during additional tourniquet application according to the present invention.

Mode(s) for Carrying Out the Invention

Hereinafter, embodiments of the present invention will be described with reference to the drawings.

<Configuration of Hemostasis Aid>

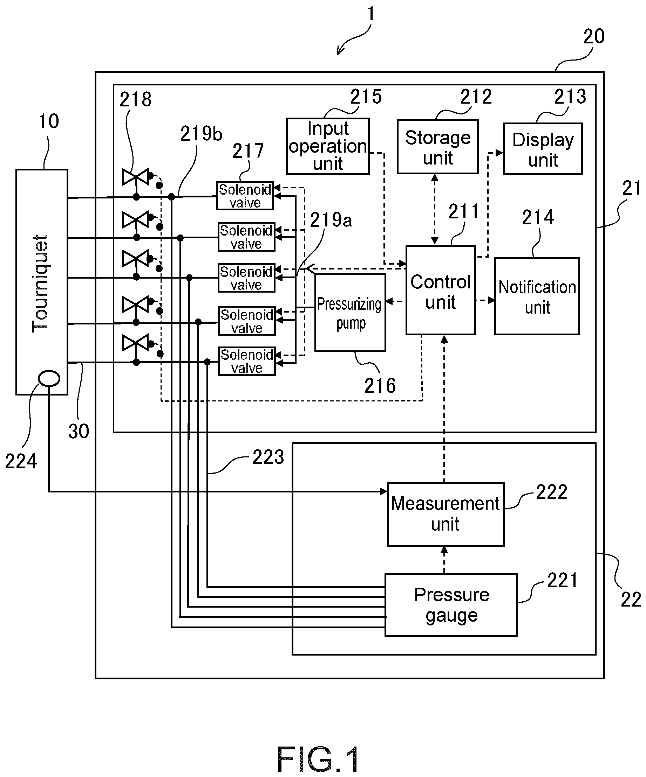

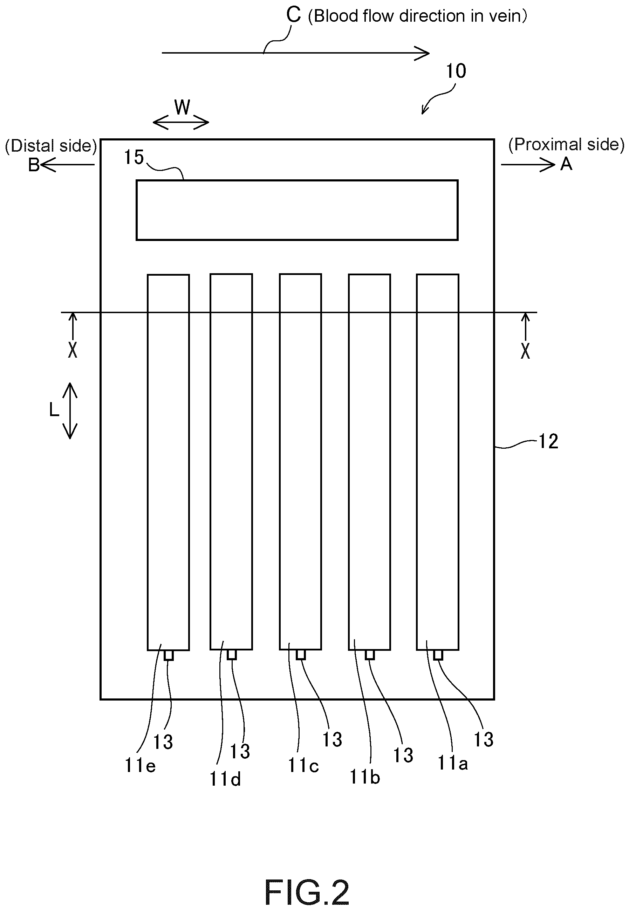

Fig. 1 is a block diagram showing a hemostasis aid according to an embodiment of the present invention. Fig. 2 is a diagram showing a configuration of a tourniquet. It should be noted that Fig. 2 is a schematic diagram showing the state of the deployed tourniquet from the top.

As shown in Fig. 1, a hemostasis aid 1 includes a tourniquet 10, a control device 20, and air tubes 30.

Hereinafter, configurations of the tourniquet 10 and the control device 20 will be described. It should be noted that the air tubes 30 are flow paths that feed a pressurizing fluid to the tourniquet 10 from the control device 20 and serves as an exhaust path of the tourniquet 10.

(Configuration of Tourniquet)

As shown in Fig. 2, the tourniquet 10 includes a plurality of, five pressurizing layers 11a to 11e divided in a width direction W of the tourniquet 10 and a band-like cover member 12 that can be wrapped around a patient's arm which is an attached site.

The plurality of pressurizing layers 11a to 11e is disposed to be adjacent to each other in the width direction W inside the cover member 12. The pressurizing layers 11a to 11e are provided with fluid introduction outlets 13, respectively.

The pressurizing layers 11a to 11e each correspond to a rubber bulb of a manchette of a traditional sphygmomanometer and are formed from materials such as a natural rubber, a synthetic rubber, silicon, a rubbery elastomer, nylon polyester, and polyurethane.

The cover member 12 has a size sufficient to accommodate the pressurizing layers 11a to 11e and is formed from materials such as cloth and nylon polyester, for example.

In Fig. 2, L denotes a length direction of the tourniquet 10 and the pressurizing layers 11a to 11e and the band-like cover member 12 actually have such a length that those can be sufficiently wrapped around at least the patient's arm in the length direction L. The cover member 12 is configured such that the tourniquet 10 is not released after the tourniquet 10 is wrapped around the patient's arm with a Magic Tape (Registered Trademark) 15 provided at one end, for example.

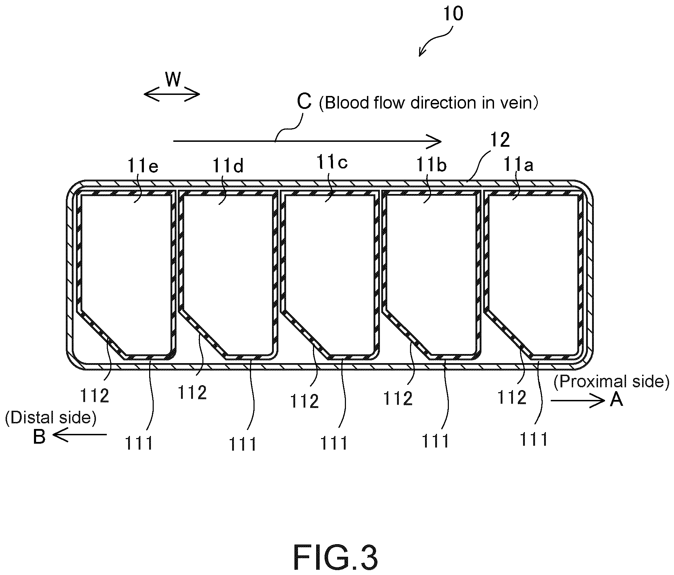

Here, a cross-sectional view taken along the line X-X of Fig. 2 is shown in Fig. 3.

In Figs. 2 and 3, the side A is the patient's proximal side (heart side) and the side B is the patient's distal side (fingertip side). Blood in a vein flows in a direction indicated by the arrow C.

Each pressurizing layer 11a to 11e includes a cutout portion 112 at a corner on the side B in a pressurizing surface 111 of the pressurizing layer 11a to 11e. That is, in each of the pressurizing layers 11a to 11e, a portion on the side B of the pressurizing surface 111 of the pressurizing layer 11a to 11e is tilted to gradually separate from the pressurizing surface as it is closer to the side B. Further, in each of the pressurizing layers 11a to 11e, the corner on the side A of the pressurizing surface 111 of the pressurizing layer 11a to 11e does not include such a cutout portion. The corner on the side A of the pressurizing surface 111 of the pressurizing layer 11a to 11e and the corner on the side B of the pressurizing surface 111 of the pressurizing layer 11a to 11e are asymmetrical.

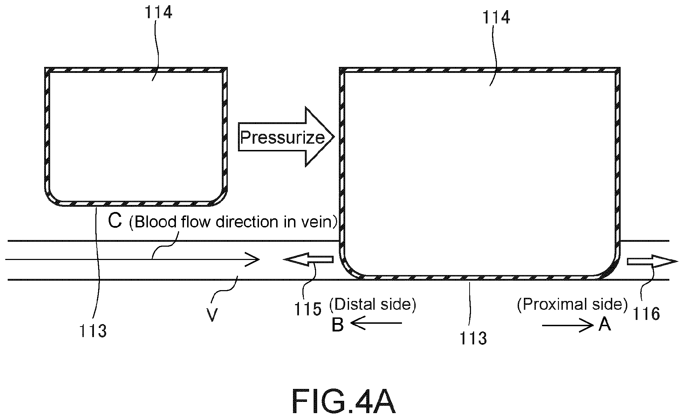

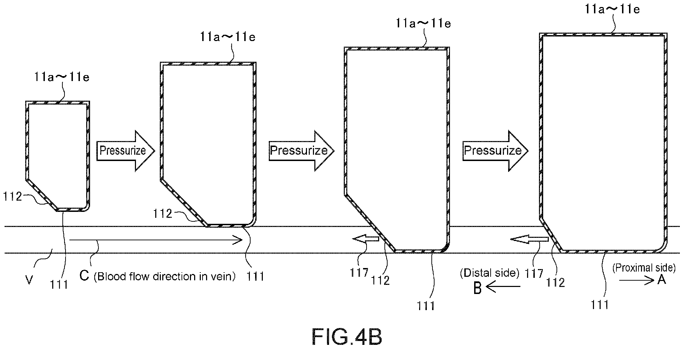

As shown in Fig. 4A, when a vein V is pressed by a pressurizing layer 114 constituted only by a flat pressurizing surface 113 from the state at the left of Fig. 4, blood in the pressed region is pushed to both sides of the side B which is the patient's distal side (side closer to hand) and the side A which the patient's proximal side (side closer to heart) (in the figure, directions indicated by the arrow 115 and the arrow 116) due to the press shown in the state at the right of Fig. 4.

In contrast, when the pressurizing layers 11a to 11e including the cutout portions 112 are pressurized from the state at the left of Fig. 4B as shown in Fig. 4B, the flat portions of the pressurizing surfaces 111 first press the vein V as shown in the state at the left of Fig. 4B and then the cutout portions 112 press the vein V as shown in the right state at the right of Fig. 4B after a delay. Therefore, when the vein V is pressed by the pressurizing layers 11a to 11e including the cutout portions 112, blood in the pressed region is more likely to be pushed to the side B which is the patient's distal side (side closer to hand) due to this press (in the figure, direction indicated by the arrow 117). That is, the backward flow rate of blood in the pressed region increases. It contributes to the distention of the blood vessel at the puncture site to be described later.

It should be noted that the cutout portion 112 may be flat, though various shapes such as a curved shape may be employed. Further, the scope of use as modifications and applications of the configurations and the like by a change in hardness by a combination of materials of the pressurizing surfaces 111 and the cutout portions 112, thickness adjustment, and the like also falls within the technical scope of the present invention. Further, in the example shown in Fig. 3, all the pressurizing layers 11a to 11e include the cutout portions 112. However, it is expected that a configuration in which at least one of the pressurizing layers 11a to 11e includes the cutout portion 112 can contribute to the distention of the blood vessel at the puncture site.

A microphone 224 for collecting Korotkov sounds is disposed in the tourniquet 10. An acoustic signal detected by the microphone 224 is transmitted to a measurement unit 222 to be described later and the Korotkov sounds are detected at the measurement unit 222.

(Configuration of Control Device)

The control device 20 feeds the pressurizing fluid to each of the pressurizing layers 11a to 11e of the tourniquet 10 via the air tube 30 in accordance with predetermined steps and also exhausts each of the pressurizing layers 11a to 11e.

The control device 20 includes a pressure control system 21 that controls the pressure of the tourniquet 10 and a blood pressure measurement system 22 that measures the patient's blood pressure via the tourniquet 10.

The pressure control system 21 includes a control unit 211, a storage unit 212, a display unit 213, a notification unit 214, an input operation unit 215, and a pressurizing pump 216.

The pressure control system 21 further includes solenoid valves 217 and exhaust valves 218 respectively corresponding to the pressurizing layers 11a to 11e.

The control unit 211 comprehensively controls operations of pressurization of the tourniquet 10 and blood pressure measurement via the tourniquet 10.

The storage unit 212 stores programs necessary for the operation of the control unit 211 and temporarily retains data and also stores the data.

The display unit 213 displays on a screen an operation procedure, measurement values, error display, preparation completion, and the like.

The notification unit 214 outputs audible sounds in case of an error, at the time of preparation completion for puncture, at the time when the tourniquet time elapses, and the like.

The input operation unit 215 includes various types of buttons and operations necessary for operation are input via the buttons. Typically, the start/end of tourniquet application or the operation of additional tourniquet application is input into the input operation unit 215.

The pressurizing pump 216 pressurizes each of the pressurizing layers 11a to 11e of the tourniquet 10 under the control of the control unit 211.

Branch paths 219a branch the flow path from the pressurizing pump 216 into flow paths 219b respectively corresponding to the pressurizing layers 11a to 11e. The air tube 30 is connected to each of the flow paths 219b and the tourniquet 10 is connected to each of the flow paths 219b via the air tubes 30.

The solenoid valves 217 and the exhaust valves 218 that operate under the control of the control unit 211 are provided on the flow paths 219b. The solenoid valves 217 regulates pressurization of the tourniquet 10 by the pressurizing pump 216.

The exhaust valves 218 each control the pressure of each of the flow paths 219b and exhausts of the fluid of each of the pressurizing layers under the control of the control unit 211.

In this embodiment, the pressurizing fluid is selectively fed to each of the pressurizing layers 11a to 11e of the tourniquet 10 via the pressurizing pump 216 and each solenoid valve 217 and each of the pressurizing layers 11a to 11e of the tourniquet 10 is selectively exhausted via each exhaust valve 218.

The blood pressure measurement system 22 includes a pressure gauge 221, the measurement unit 222, and flow paths 223.

The pressure gauge 221 is connected to each of the flow paths 219b in communication with the pressurizing layers 11a to 11e via the flow paths 223. The measurement unit 222 measures a pressure (patient's blood pressure) of each of the pressurizing layers 11a to 11e of the tourniquet 10 on the basis of a value measured by the pressure gauge 221. Data on the patient's blood pressure measured by the measurement unit 222 is transmitted to the control unit 211.

(Operation of Hemostasis Aid)

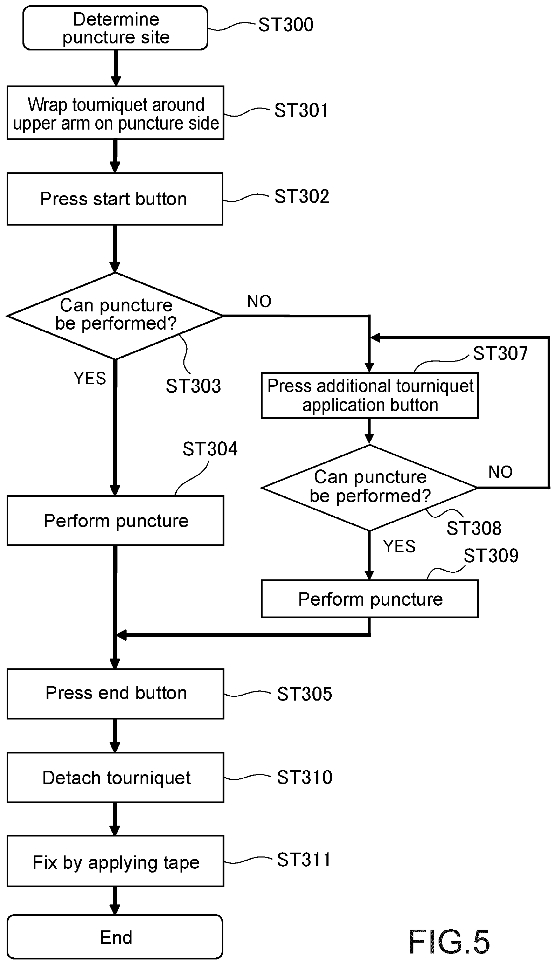

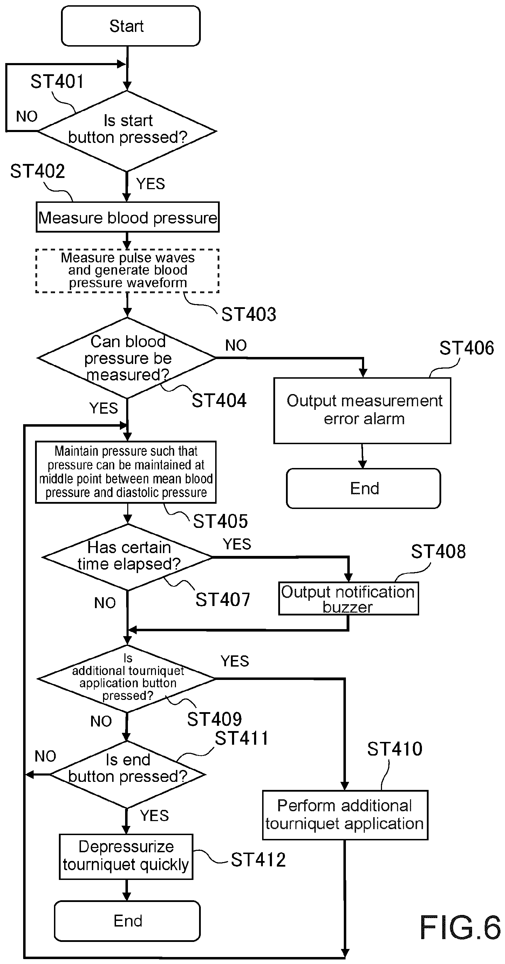

Fig. 5 is a flowchart showing an operation on the hemostasis aid 1. Fig. 6 is a flowchart showing an operation of the hemostasis aid 1 in connection with such an operation.

A person who performs puncture determines a puncture site of the patient (ST300) and wraps the tourniquet 10 around the patient's upper arm on a puncture side (ST301). The person who performs puncture presses a start button of the input operation unit 215 (ST302).

When the start button is pressed (ST401), the hemostasis aid 1 measures a blood pressure (ST402) and measures pulse waves and generates a blood pressure waveform (ST403).

- Measurement of Blood Pressure (ST402)

The blood pressure measurement is performed by detecting a change in pressure of the pressurizing layer 11a and Korotkov sounds. A pressure when Korotkov sounds can be sensed in a process of boosting the pressure of the pressurizing layer 11a via the pressurizing pump 216 will be referred to as a diastolic pressure. A pressure when the Korotkov sounds disappear in a process of further boosting the pressure will be referred to as a systolic pressure.

It should be noted that the diastolic pressure and the systolic pressure may be detected by another blood pressure measurement method such as an oscillometric method.

- Pulse Wave Measurement and Blood Pressure Waveform (ST403)

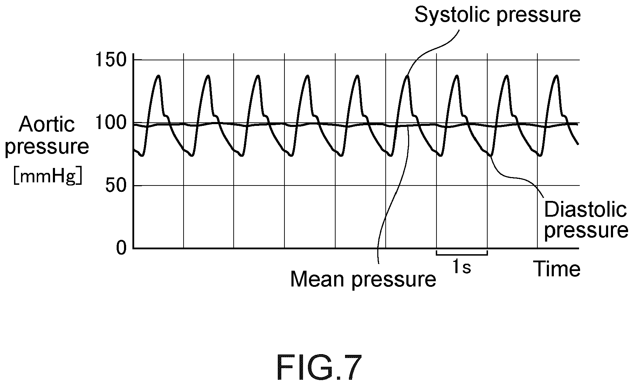

At the same time when the blood pressure is measured, the pressure gauge 221 measures a pressure waveform (hereinafter, referred to as pulse waves). While the tourniquet pressure is maintained, the hemostasis aid 1 continuously measures pulse waves on the basis of a pressure obtained by the blood pressure measurement system 22 via the tourniquet 10. The measured pulse waves are corrected in the following manner on the basis of the blood pressure measured in the blood pressure measurement. The highest value of pulse waves is set as the systolic pressure and the lowest value is set as the diastolic pressure (see Fig. 7). For example, the correction is performed as systolic pressure 140/diastolic pressure 70. Here, the corrected waveform is set as the blood pressure waveform.

Accordingly, it is possible to perform continuous blood pressure measurement without measuring a blood pressure multiple times and to cope with the patient's condition change in case of emergency or the like.

- Optimization of Tourniquet Pressure

The hemostasis aid 1 has a function of storing information regarding pulse waves and a blood pressure waveform in the storage unit 212 when measuring a blood pressure. The control unit 211 continuously generates a blood pressure waveform on the basis of the pressure obtained by the blood pressure measurement system 22 and the information stored in the storage unit 212 while the tourniquet pressure is maintained. The hemostasis aid 1 is capable of maintaining a suitable tourniquet pressure in accordance with a change in blood pressure obtained on the basis of the blood pressure waveform. That is, the control unit 211 captures fluctuations of the blood pressure and controls the tourniquet 10 at an optimal pressure.

The control unit 211 recognizes a change in blood pressure and regulates the pressure such that the pressure of the tourniquet 10 is constantly maintained to a mean value (target pressure) of a blood pressure mean value and the diastolic pressure.

The optimal pressure is typically a value determined in the following manner.

That is, the blood pressure mean value is determined in accordance with (systolic pressure + diastolic pressure)/2.

Then, the value is determined in accordance with (blood pressure mean value + diastolic pressure)/2. It is an optimal tourniquet pressure for each of the pressurizing layers 11a to 11e. Accordingly, it is unnecessary to boost the pressure by applying unnecessary pressure, which imposes a burden on the patient, unlike traditional blood pressure measurement.

The control unit 211 controls the pressurizing pump 216 and the exhaust valves 218 to constantly maintain the optimal pressure on the basis of a change in blood pressure waveform obtained from the blood pressure measurement system 22.

Also, continuously monitoring the blood pressure waveform makes it possible to find an irregular pulse. With a configuration to notify of an irregular pulse when it occurs, it can be helpful during puncture or in case of sudden change in patient's condition (in case of emergency). It should be noted that when pulse waves cannot be detected, the control unit 211 may control the pressurizing pump 216 and the exhaust valves 218 to maintain a fixed pressure without following fluctuations of the blood pressure. When pulse waves cannot be detected, it can be caused by a failure, high pressure or low pressure, a sudden change in patient, or the like. When pulse waves cannot be detected, an alarm may be output.

The control unit 211 is capable of measuring a blood pressure. When the optimal pressure is determined by calculation (ST404), the control unit 211 first boosts the pressure of the pressurizing layer 11a on the patient's proximal side A to the optimal pressure and also pressurizes the other pressurizing layers 11b to 11e sequentially (in the order of 11b, 11c, 11d, and 11e) while maintaining the pressure (ST405). The blood pressure measurement system 22 measures a pressure of each of the pressurizing layers 11a to 11e sequentially and notifies, when all the pressurizing layers 11a to 11e have the optimal pressure, the person who performs puncture of it by a notification sound.

It should be noted that the control unit 211 outputs, in a case where the blood pressure cannot be measured in Step 404, a measurement error alarm through the notification unit 214 (Step 406). At that time, the control unit 211 may perform display on the display unit 213 to instructing to replace the tourniquet 10.

In a case where the person who performs puncture judges that the blood vessel at the patient's puncture position is sufficiently distended and the puncture can be performed (Step 303), the person who performs puncture performs puncture (Step 304), presses, when the puncture is completed, the end button of the input operation unit 215 (Step 305), detaches the tourniquet 10 (Step 310), and fixes the area of the puncture position by applying a tape (Step 311). It should be noted that such a sequence of puncture may be automatically performed by a puncture system proposed by the inventors of the present invention (see Japanese Patent Application No. 2017-113581 and the like).

On the other hand, in a case where the person who performs puncture judges that the blood vessel at the patient's puncture position is insufficiently distended and the puncture cannot be performed, the person who performs puncture presses an additional tourniquet application button of the input operation unit 215 (Step 307). In a case where the person who performs puncture judges that the puncture can be performed this time (Step 308), the person who performs puncture performs puncture (Step 309), presses, when the puncture is completed, the end button of the input operation unit 215 (Step 305), detaches the tourniquet 10 (Step 310), and fixes the area of the puncture position by applying a tape (Step 311).

In a case where the person who performs puncture judges that the blood vessel at the patient's puncture position is still insufficiently distended and the puncture cannot be performed even after the additional tourniquet application, the person who performs puncture presses the additional tourniquet application button of the input operation unit 215 again (Step 307), performs puncture (Step 309), presses, when the puncture is completed, the end button of the input operation unit 215 (Step 305), detaches the tourniquet 10 (Step 310), and fixes the area of the puncture position by applying a tape (Step 311).

After pressurizing the pressurizing layers 11a to 11e in the hemostasis aid 1 (Step 405), the person who performs puncture normally finishes the puncture and presses the end button of the input operation unit 215 or presses the additional tourniquet application button of the input operation unit 215 as described above. However, the person who performs puncture leaves it without performing those operations in some cases. In a case where the person who performs puncture does not press the end button of the input operation unit 215 and the additional tourniquet application button (Step 407) after a certain time elapses after pressurizing the pressurizing layers 11a to 11e (Step 405), the control unit 211 outputs a notification buzzer through the notification unit 214 (Step 408) to prompt the person who performs puncture to continue the operation. It should be noted that it may be possible to arbitrarily set the operation time or buzzer sound of the notification buzzer.

Otherwise, in a case where the person who performs puncture presses the additional tourniquet application button of the input operation unit 215 (Step 409), the control unit 211 performs the operation of additional tourniquet application (Step 410). That is, in Step 410, the control unit 211 controls the pressurizing pump 216 and the exhaust valves 218, first boosts the pressure of the pressurizing layer 11a on the patient's proximal side A to the optimal pressure again, and also pressurizes the other pressurizing layers 11b to 11e sequentially (in the order of 11b, 11c, 11d, and 11e) while maintaining the pressure.

It should be noted that the additional tourniquet application is performed at the optimal pressure, though arbitrary pressure may be added setting the systolic pressure as the upper limit. Further, in a case of performing the additional tourniquet application because of insufficient distention of the blood vessel, the blood pressure measurement may be performed again, the pulse waves may be corrected, and the blood pressure waveform may be generated.

In a case where the person who performs puncture presses the end button of the input operation unit 215 (Step 411), the control unit 211 quickly depressurizes the tourniquet 10 (Step 412). That is, in Step 412, the control unit 211 controls the pressurizing pump 216 and the exhaust valves 218 and depressurizes the pressurizing layers 11a to 11.

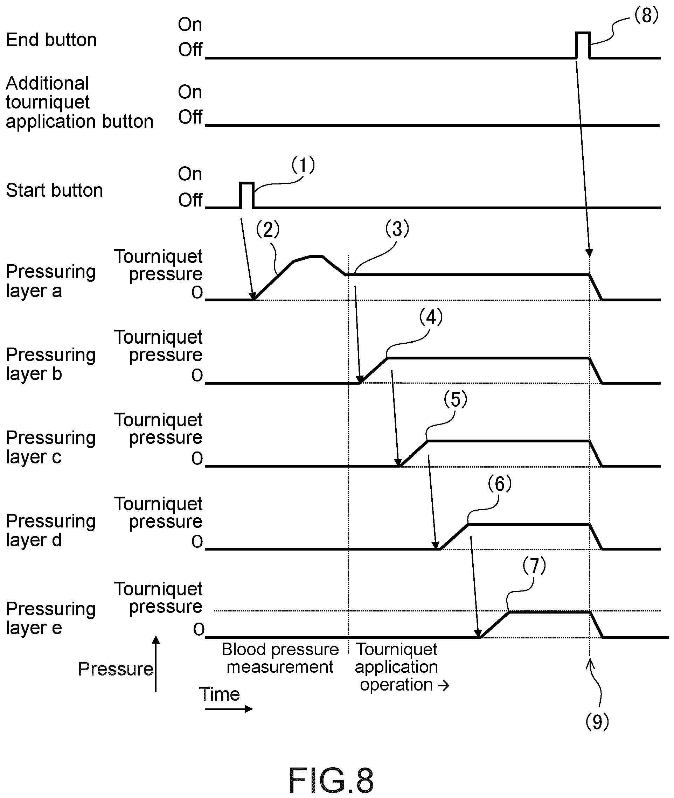

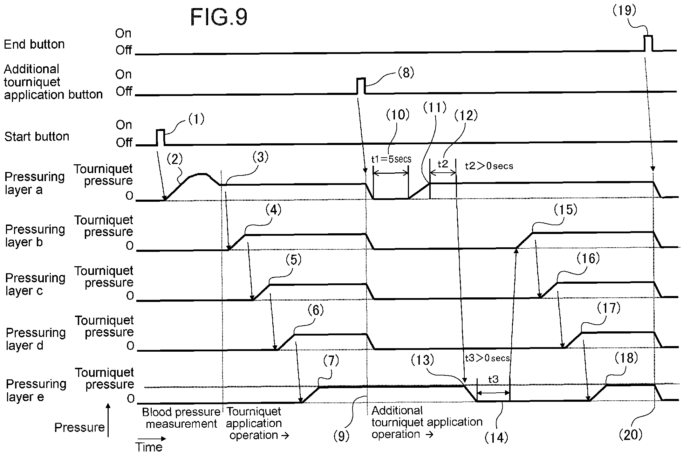

Figs. 8 and 9 are timing charts showing timings of pressurizing the pressurizing layers 11a to 11e in response to the above-mentioned operations on the input operation unit 215 by the person who performs puncture. Fig. 6 shows a case where the person who performs puncture presses the start button and presses the end button without pressing the additional tourniquet application button. Fig. 7 shows a case where the person who performs puncture presses the start button, presses the additional tourniquet application button, and presses the end button.

First of all, the case where the person who performs puncture presses the start button and presses the end button without pressing the additional tourniquet application button will be described.

As shown in Fig. 8, the person who performs puncture presses the start button of the input operation unit 215 and the control unit 211 detects it (Fig. 8 (1)) and performs control to pressurize the pressurizing layer 11a of tourniquet 10 in order to determine the tourniquet pressure that is the optimal pressure (Fig. 8 (2)).

The control unit 211 determines the tourniquet pressure that is the optimal value and performs control to pressurize the pressurizing layer 11a already in the pressurized state at the optimal tourniquet pressure (Fig. 8 (3)). By using the pressurizing layer 11a in order to determine the tourniquet pressure, it is possible to continuously pressurize the pressurizing layer without temporarily depressurizing the pressurizing layer into the initial state for the tourniquet application operation after pressurizing the pressurizing layer in order to determine the tourniquet pressure. Therefore, the time until the start of the tourniquet application operation can be shortened.

Next, the control unit 211 performs control to start pressurizing the pressurizing layer 11b at the tourniquet pressure after the target pressure is reached after starting pressurizing the pressurizing layer 11a at the tourniquet pressure (Fig. 8 (4)).

Next, after the target pressure is reached after starting pressurizing the pressurizing layer 11b at the tourniquet pressure, the control unit 211 performs control to start pressurizing the pressurizing layer 11c at the tourniquet pressure (Fig. 8 (5)).

Next, the control unit 211 performs control to start pressurizing the pressurizing layer 11d at the tourniquet pressure after the target pressure is reached after starting pressurizing the pressurizing layer 11c at the tourniquet pressure (Fig. 8 (6)).

Next, the control unit 211 performs control to start pressurizing the pressurizing layer 11e at the tourniquet pressure after the target pressure is reached after starting pressurizing the pressurizing layer 11d at the tourniquet pressure (Fig. 8 (7)).

It should be noted that pressurizing a next pressurizing layer may be started right before the target pressure is reached for the purpose of time saving.

In the above-mentioned manner, all the pressurizing layers 11a to 11e of the tourniquet 10 are pressurized at the tourniquet pressure.

In this state, the person who performs puncture performs puncture and presses the end button of the input operation unit 215 when the puncture is completed. When the control unit 211 detects it (Fig. 8 (8)), the control unit 211 performs control to depressurize all the pressurizing layers 11a to 11e of the tourniquet 10 to have the initial pressure (Fig. 8 (9)).

Next, the case where the person who performs puncture presses the start button, presses the additional tourniquet application button, and presses the end button will be described.

As shown in Fig. 9, when the person who performs puncture presses the start button of the input operation unit 215, the control unit 211 detects it (Fig. 9 (1)) and performs control to pressurize the pressurizing layer 11a of tourniquet 10 in order to determine the tourniquet pressure that is the optimal pressure (Fig. 9 (2)).

The control unit 211 determines the tourniquet pressure that is the optimal value and performs control to pressurize the pressurizing layer 11a is already in the pressurized state at the optimal tourniquet pressure (Fig. 9 (3)).

Next, after the target pressure is reached after starting pressurizing the pressurizing layer 11a at the tourniquet pressure, the control unit 211 performs control to start pressurizing the pressurizing layer 11b at the tourniquet pressure (Fig. 9 (4)).

Next, after the target pressure is reached after starting pressurizing the pressurizing layer 11b at the tourniquet pressure, the control unit 211 performs control to start pressurizing the pressurizing layer 11c at the tourniquet pressure (Fig. 9 (5)).

Next, after the target pressure is reached after starting pressurizing the pressurizing layer 11c at the tourniquet pressure, the control unit 211 performs control to start pressurizing the pressurizing layer 11d at the tourniquet pressure (Fig. 9 (6)).

Next, after the target pressure is reached after starting pressurizing the pressurizing layer 11d at the tourniquet pressure, the control unit 211 performs control to start pressurizing the pressurizing layer 11e at the tourniquet pressure (Fig. 9 (7)).

In the above-mentioned manner, all the pressurizing layers 11a to 11e of the tourniquet 10 are pressurized at the tourniquet pressure.

In this state, in a case where the person who performs puncture judges that the blood vessel is insufficiently distended and the puncture cannot be performed, the person who performs puncture presses the additional tourniquet application button of the input operation unit 215. When the control unit 211 detects it (Fig. 9 (8)), the control unit 211 performs control to temporarily depressurize the other pressurizing layers 11a to 11d to have the initial pressure while maintaining the pressurized state of the pressurizing layer 11e of the tourniquet 10 (Fig. 9 (9)). After a predetermined time t1 (e.g., t1 = about 5 seconds) elapses (Fig. 9 (10)), the control unit 211 performs control to pressurize the pressurizing layer 11a at the tourniquet pressure again (Fig. 9 (11)). By providing the predetermined time t1, it is possible to cause sufficient blood to flow in under the pressurizing layers 11a to 11d. The predetermined time t1 may be variable as appropriate.

After a predetermined time t2 (t2 > 0 seconds, Fig. 9 (12)) elapses after the control unit 211 starts pressurizing the pressurizing layer 11a at the tourniquet pressure again, the control unit 211 performs control to depressurize the pressurizing layer 11e to have the initial pressure (Fig. 9 (13)). By providing the predetermined time t2, it is possible to collect sufficient blood under the pressurizing layers 11a to 11e.

At this time, the pressurizing layer 11a is in the pressurized state and the other pressurizing layers 11b to 11e have the initial pressure at which those are depressurized. Accordingly, it is possible to cause blood to flow in directly under the tourniquet 10.

Next, after a predetermined time t3 (t3 > 0 seconds) elapses (Fig. 9 (14)) after the control unit 211 depressurizes the pressurizing layer 11e to have the initial pressure, the control unit 211 performs control to start pressurizing the pressurizing layer 11b at the tourniquet pressure (Fig. 9 (15)).

Next, after the predetermined time elapses after the target pressure is reached after the control unit 211 starts pressurizing the pressurizing layer 11b at the tourniquet pressure, the control unit 211 performs control to start pressurizing the pressurizing layer 11c at the tourniquet pressure (Fig. 9 (16)).

Next, after the target pressure is reached after the control unit 211 starts pressurizing the pressurizing layer 11c at the tourniquet pressure, the control unit 211 performs control to start pressurizing the pressurizing layer 11d at the tourniquet pressure (Fig. 9 (17)).

Next, after the target pressure is reached after the control unit 211 starts pressurizing the pressurizing layer 11d at the tourniquet pressure, the control unit 211 performs control to start pressurizing the pressurizing layer 11e at the tourniquet pressure (Fig. 9 (18)).

In the above-mentioned manner, all the pressurizing layers 11a to 11e of the tourniquet 10 are pressurized at the tourniquet pressure.

In this state, the person who performs puncture performs puncture, and presses the end button of the input operation unit 215 when the puncture is completed. When the control unit 211 detects it (Fig. 9 (19)), the control unit 211 performs control to depressurize all the pressurizing layers 11a to 11e of the tourniquet 10 to have the initial pressure (Fig. 9 (20)).

Since in the hemostasis aid 1 configured as described above, the configuration in which the pressurizing layers 11b to 11e of the tourniquet 10 are pressurized sequentially from the pressurizing layer 11a on the patient's proximal side A to the patient's distal side B is employed, the blood vessel on the puncture position (puncture site) located on the patient's distal side B with respect to the tourniquet 10 can be more reliably distended.

Further, since the hemostasis aid 1 has the additional tourniquet application mode such that the tourniquet 10 can be continuously pressurized in a similar manner again, the blood vessel on the puncture position (puncture site) can be more reliably distended without taking much time.

Therefore, with the hemostasis aid 1, the puncture can be more reliably performed for a patient having difficulties in puncture because of insufficient distended blood vessels. Further, with the hemostasis aid 1, an increase in success rate (reduction in failure) by a beginner at puncture can be achieved.

<Another Configuration Example 1 of Hemostasis Aid>

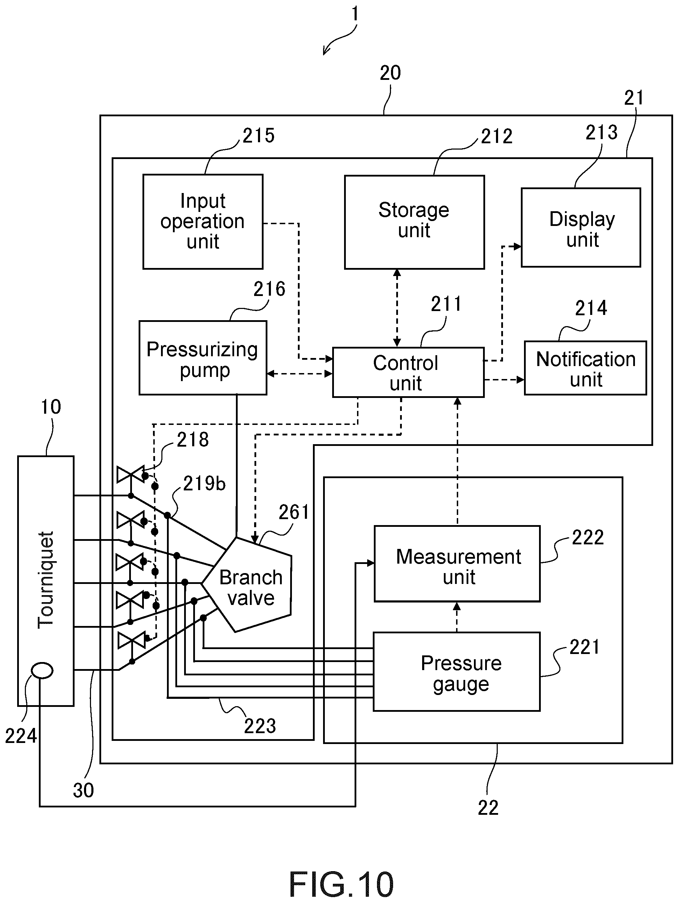

Fig. 10 is a diagram showing a configuration of a hemostasis aid according to another embodiment of the present invention.

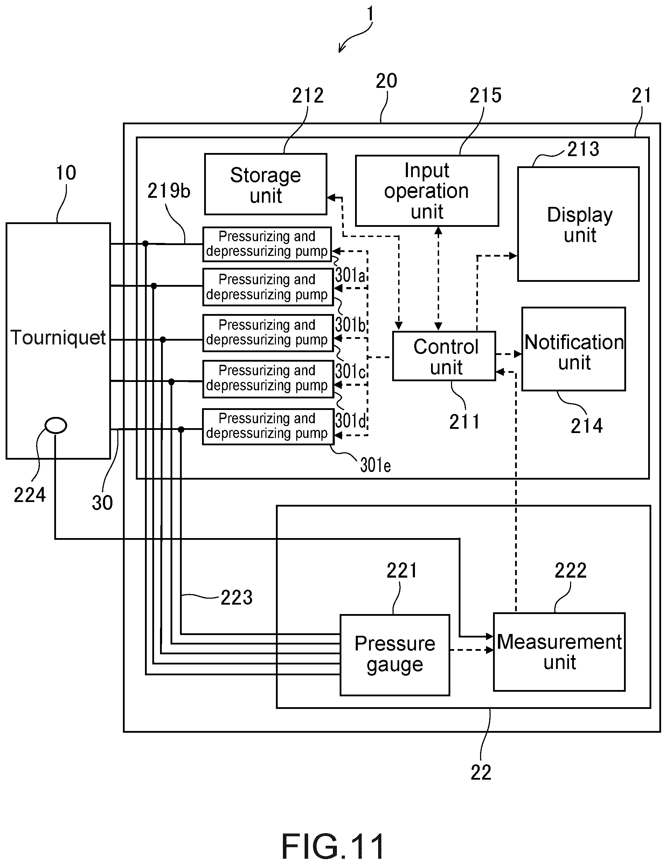

In the hemostasis aid 1 shown in Fig. 10, elements similar to those of the hemostasis aid shown in Fig. 11 will be denoted by identical reference signs and descriptions thereof will be omitted.

As shown in Fig. 10, a control device 20 of the hemostasis aid 1 includes a branch valve 261 using both branch paths that branch the pressurizing fluid into each of the pressurizing layers 11a to 11e from the pressurizing pump 216 and solenoid valves that open and close the flow paths branched by the branch paths. That is, this branch valve 261 is a multi-directional solenoid valve that branches the pressurizing fluid into the pressurizing layers 11a to 11e from the pressurizing pump 216 and opens and closes each of the branched flow paths.

The control unit 211 controls open/close of the branch valve 261.

With the hemostasis aid 1 configured as described above, since the number of pumps can be reduced as in the hemostasis aid shown in Fig. 11, cost saving and downsizing can be achieved and a reduction in number of components such as the solenoid valves can also be achieved by employing the branch valve 261.

<Another Configuration Example 2 of Hemostasis Aid>

Fig. 11 is a diagram showing a configuration of a hemostasis aid according to still another embodiment of the present invention.

In the hemostasis aid 1 shown in Fig. 11, elements similar to those of the hemostasis aid shown in Fig. 11 will be denoted by identical reference signs and descriptions thereof will be omitted.

As shown in Fig. 11, a control device 20 of the hemostasis aid 1 includes pressurizing and depressurizing pumps 301a to 301e for pressurizing and depressurizing each of the pressurizing layers 11a to 11e. The pressurizing and depressurizing pumps 301a to 301e are provided for the pressurizing layers 11a to 11e, respectively.

The control unit 211 controls operations of the pressurizing and depressurizing pumps 301a to 301e.

With the hemostasis aid 1 configured as described above, since the pressurizing and depressurizing pumps constitute a pressurizing and depressurizing mechanical system, the hemostasis aid 1 can be constituted by versatile components and it is expected that the hemostasis aid 1 has good accuracy and is less likely to malfunction.

<Another Configuration Example 3 of Hemostasis Aid>

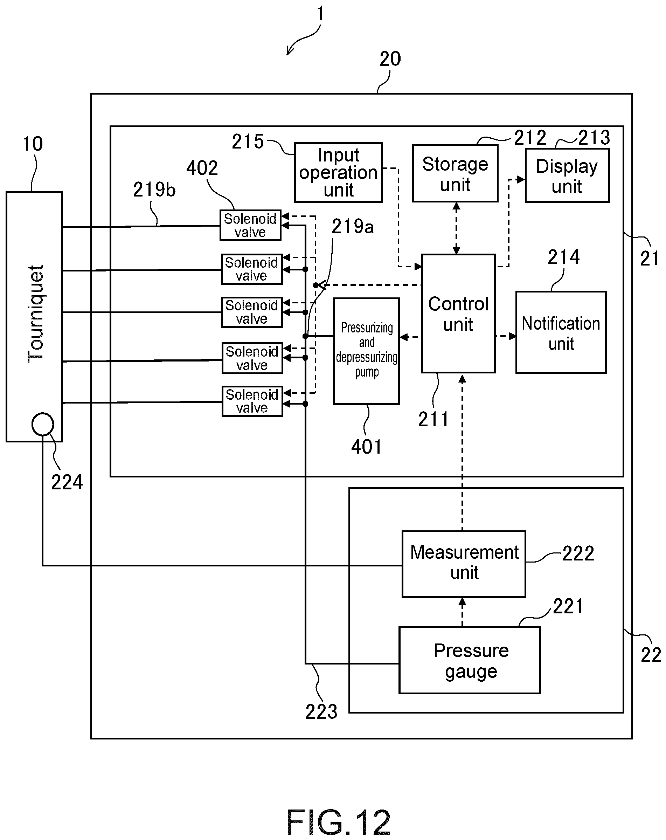

Fig. 12 is a diagram showing a configuration of a hemostasis aid according to still another embodiment of the present invention.

In the hemostasis aid 1 shown in Fig. 12, elements similar to those of the hemostasis aid shown in Fig. 11 will be denoted by identical reference signs and descriptions thereof will be omitted.

As shown in Fig. 12, a control device 20 of the hemostasis aid 1 includes an pressurizing and depressurizing pump 401 and solenoid valves 402 for regulating the degree of pressurizing and depressurizing for each of the pressurizing layers 11a to 11e.

The control unit 211 controls the operation of the pressurizing and depressurizing pump 401 and the opening of each of the solenoid valves 402.

With the hemostasis aid 1 configured as described above, since the pressurizing and depressurizing pump 401 and the solenoid valves 402 constitute a pressurizing and depressurizing mechanical system, the hemostasis aid 1 can be constituted by versatile components and it is expected that the hemostasis aid 1 has good accuracy and is less likely to malfunction.

<Other Operation Examples of Hemostasis Aid>

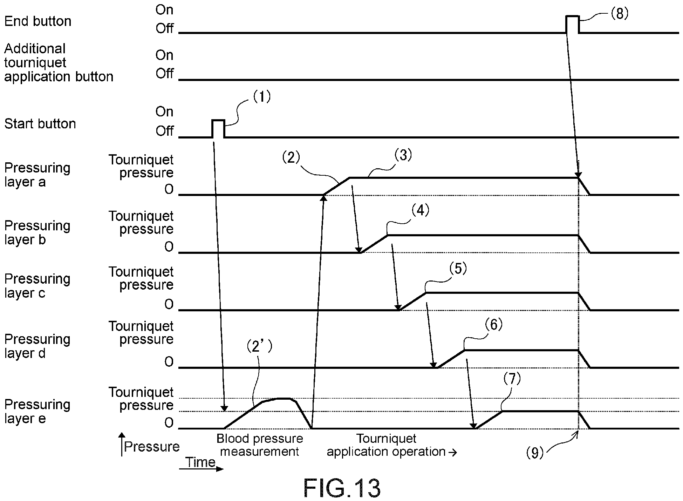

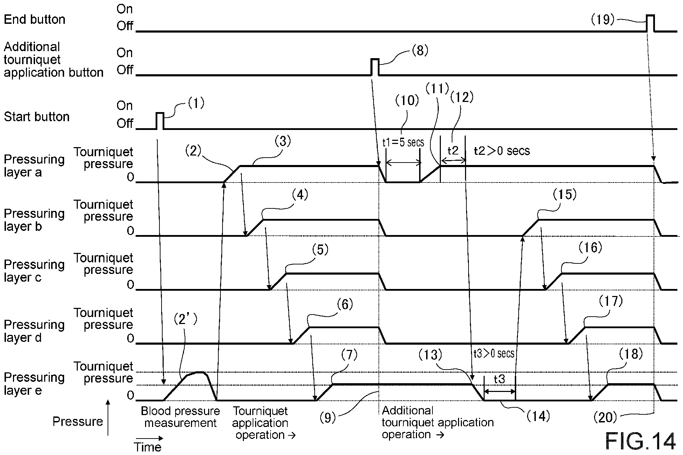

Figs. 13 and 14 are timing charts according to an embodiment different from that of the timings of pressurizing the pressurizing layers shown in Figs. 8 and 9. Fig. 13 shows a case where the person who performs puncture presses the start button and presses the end button without pressing the additional tourniquet application button. Fig. 14 shows a case where the person who performs puncture presses the start button, presses the additional tourniquet application button, and presses the end button.

In the embodiment shown in Figs. 8 and 9, the pressurizing layer 11a on the patient's proximal side A out of the pressurizing layers 11a to 11e of the tourniquet 10 is pressurized and the pressure of the pressurizing layer 11a is measured in order to determine the tourniquet pressure that is the optimal pressure (Fig. 8 (2), Fig. 9 (2)). This embodiment is different from the embodiment shown in Figs. 8 and 9 in that the pressurizing layer 11e on the patient's distal side B out of the pressurizing layers 11a to 11e of the tourniquet 10 is pressurized and its pressure is measured in order to determine the tourniquet pressure that is the optimal pressure (Fig. 13 (2'), Fig. 14 (2')).

It is expected that there is a possibility that a blood pressure at a position much closer to the puncture site can be measured by measuring the pressurizing layer 11e on the patient's distal side B.

However, the present invention is not limited thereto, the blood pressure may be measured by using any pressurizing layer of the pressurizing layers 11a to 11e of the tourniquet 10 in order to determine the tourniquet pressure that is the optimal pressure.

<Modified Example (Part I) of Tourniquet>

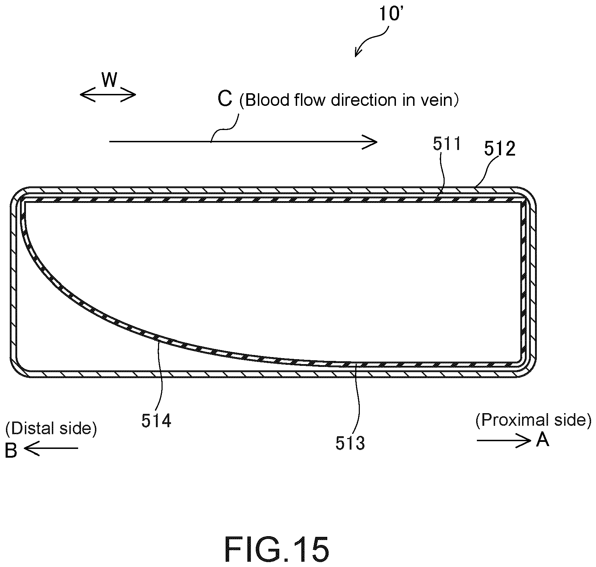

Fig. 15 is a cross-sectional view showing a modified example (Part I) of the tourniquet according to the present invention.

As shown in Fig. 15, a tourniquet 10' includes a pressurizing member 511 and a band-like cover member 512 that accommodates the pressurizing member 511 and can be wrapped around the patient's arm which is the attached site.

The pressurizing member 511 is provided with a fluid introduction outlet (not shown in the figure).

The pressurizing member 511 is, as in the above-mentioned embodiment, formed from materials such as a natural rubber, a synthetic rubber, silicon, a rubbery elastomer, nylon polyester, and polyurethane.

The cover member 512 has a size sufficient to accommodate the pressurizing member 511 and is formed from materials such as cloth and nylon polyester, for example.

It should be noted that the pressurizing member 511 and the band-like cover member 512 have such a length that those can be sufficiently wrapped around at least the patient's arm in the length direction. The cover member 512 is configured such that the tourniquet 10' is not released after the tourniquet 10' is wrapped around the patient's arm with a Magic Tape (Registered Trademark) (not shown in the figure) provided at one end, for example.

Here, the pressurizing member 511 includes a cutout portion 514 at a corner on the side B in a pressurizing surface 513 of the pressurizing member. Although the surface of the cutout portion 514 has a curved surface shape as shown in the figure, it may have another shape such as a flat shape.

Although not shown in the figure, a control device of this tourniquet 10' only needs to feed a pressurizing fluid to the pressurizing member 511 of this tourniquet 10' and exhausts of the pressurizing member 511 as minimum functions.

When the pressurizing member 511 including the cutout portion 514 as described above is pressurized, the flat portion of the pressurizing surface 513 first presses a vein, and then the cutout portion 514 presses the vein after a delay.

That is, also in this tourniquet 10', the vein portion compressed by this tourniquet 10' expands in a direction opposite to the direction in which the blood flows. Accordingly, blood flowing into this region is compressed in the vein, flows backward, and joins blood flowing from the patient's distal side, and the vein blood vessel is greatly distended. Therefore, the blood vessel at the puncture site can be reliably distended also with this tourniquet 10'.

It should be noted that the hemostasis aid using this tourniquet 10' may have the blood pressure measurement function and the tourniquet pressure control function via the tourniquet, the tourniquet pressure maintaining function, and the like as in the above-mentioned embodiment.

<Modified Example (Part II) of Tourniquet>

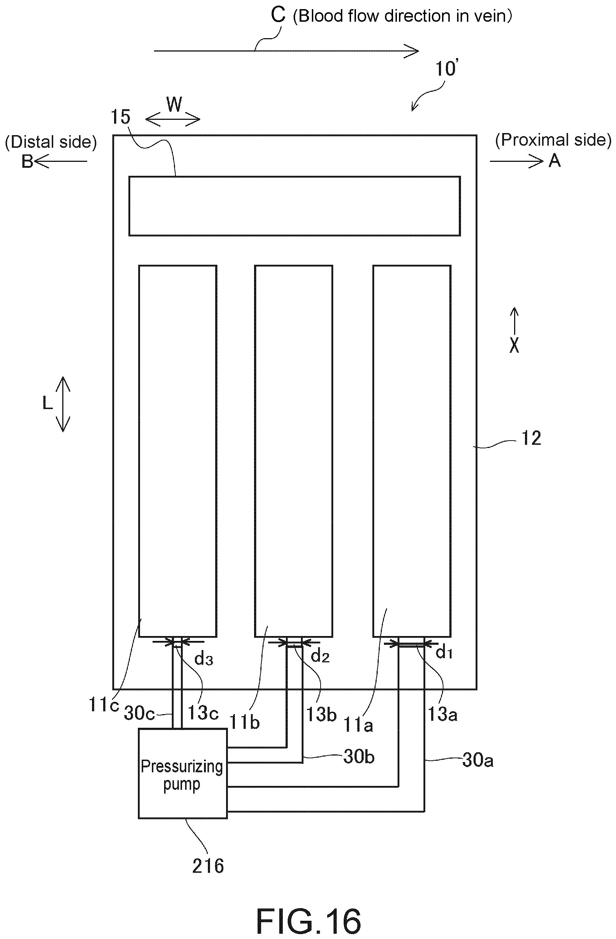

Fig. 16 is a schematic plan view showing a tourniquet according to a modified example (Part II) of the present invention.

As shown in Fig. 16, a tourniquet 10' includes three pressurizing layers 11a to 11c having the same capacity, for example, which are divided in a width direction W of the tourniquet 10' and a band-like cover member 12 that can be wrapped around the patient's arm which is the attached site. It should be noted that the pressurizing layers 11a to 11c may be integrated with the cover member 12. The main part of the tourniquet 10' can be configured by bonding two sheets in upper and lower directions such that spaces corresponding to the pressurizing layers 11a to 11c can be formed, for example.

The pressurizing layers 11a to 11c are disposed to be adjacent to each other in the width direction W inside the cover member 12 and the pressurizing layers 11a to 11c are provided with fluid introduction outlets 13a to 13c, respectively.

The fluid introduction outlets 13a to 13c have diameters gradually smaller sequentially from the pressurizing layer 11a on the proximal side A which is one end side in the width direction to the pressurizing layer 11c on the distal side B which is the other end side. That is, assuming that the diameter of the fluid introduction outlet 13a is denoted by $d_1$, the diameter of the fluid introduction outlet 13b is denoted by $d_2$, and the diameter of the fluid introduction outlet 13c is denoted by $d_3$, their relationship is as follows

$$d_1 > d_2 > d_3.$$

For the use, for example, the single pressurizing pump 216 supplies pressurizing gas into each of the pressurizing layers 11a to 11c via air tubes 30a to 30c and the fluid introduction outlets 13a to 13c. The inner diameters of the air tubes 30a to 30c are set in a relationship similar to that of the diameters of the fluid introduction outlets 13a to 13c. That is, assuming that the inner diameters of the air tubes 30a to 30c are denoted by $d_{IN1}$, $d_{IN2}$, $d_{IN3}$, respectively, their relationship is as follows

$$d_{IN1} > d_{IN2} > d_{IN3}.$$

In this case, the pressure of the pressurizing gas into each of the pressurizing layers 11a to 11c is favorably constant. It means that the flow rate of the pressurizing gas into the pressurizing layers 11a to 11c is in the relationship of pressurizing layer 11a > pressurizing layer 11b > pressurizing layer 11c. Accordingly, it is possible to sequentially delay the inflation of the pressurizing layers 11a to 11c and inflate the pressurizing layer 11a, the pressurizing layer 11b, and the pressurizing layer 11c in the stated order.

That is, with the tourniquet 10' having such a configuration, the pressurizing layer 11a first inflates, the pressurizing layer 11b subsequently inflates, and the pressurizing layer 11c finally inflates. That is, the region pressurized by the tourniquet 10' can be configured to expand from the proximal side A which is the one end side in the width direction W of the tourniquet 10' to the distal side B which is the other end side. In addition, the control mechanism and the like are unnecessary, and the action and effect according to the present invention can be realized with a simple configuration of the tourniquet 10', the pressurizing pump 216, and the air tubes 30a to 30c according to this embodiment.

<Modified Example (Part III) of Tourniquet>

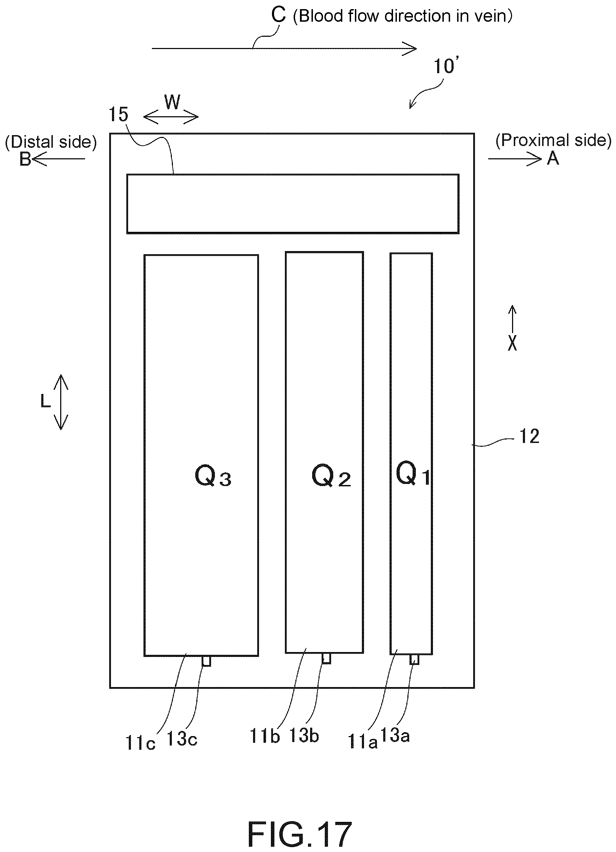

Fig. 17 is a schematic plan view showing a tourniquet according to a modified example (Part III) of the present invention.

As shown in Fig. 17, a tourniquet 10' includes three pressurizing layers 11a to 11c divided in a width direction W of the tourniquet 10', for example, and a band-like cover member 12 that can be wrapped around the patient's arm which is the attached site.

The pressurizing layers 11a to 11c are disposed to be adjacent to each other in the width direction W inside the cover member 12 and the pressurizing layers 11a to 11c are provided with the fluid introduction outlets 13a to 13c, respectively.

The pressurizing layers 11a to 11c have capacity gradually larger sequentially from the pressurizing layer 11a on the proximal side A which is one end side in the width direction W to the pressurizing layer 11c on the distal side B which is the other end side. That is, assuming that the capacity of the pressurizing layer 11a is denoted by $Q_1$, the capacity of the pressurizing layer 11b is denoted by $Q_2$, and the capacity of the pressurizing layer 11c is denoted by $Q_3$, their relationship is as follows

$Q_1 < Q_2 < Q_3$. The capacity can be regulated typically by changing the width of the pressurizing layer 11a to 11c.

For the use, for example, a single pressurizing pump (not shown in the figure) supplies pressurizing gas into the pressurizing layers 11a to 11c via air tubes (not shown in the figure) and the fluid introduction outlets 13a to 13c. In this case, the flow rate of the pressurizing gas into each of the pressurizing layers 11a to 11c is favorably constant. Accordingly, it is possible to sequentially delay the inflation of the pressurizing layers 11a to 11c and inflate the pressurizing layer 11a, the pressurizing layer 11b, and the pressurizing layer 11c in the stated order.

That is, with the tourniquet 10' having such a configuration, the pressurizing layer 11a first inflates with sufficient tension, the pressurizing layer 11b subsequently inflates with sufficient tension, and the pressurizing layer 11c finally inflates with sufficient tension. That is, the region pressurized by the tourniquet 10' can be configured to expand from the proximal side A which is the one end side in the width direction W of the tourniquet 10' to the distal side B which is the other end side. In addition, the control mechanism and the like are unnecessary, and the action and effect according to the present invention can be realized with a simple configuration.

<Modified Example (Part IV) of Tourniquet>

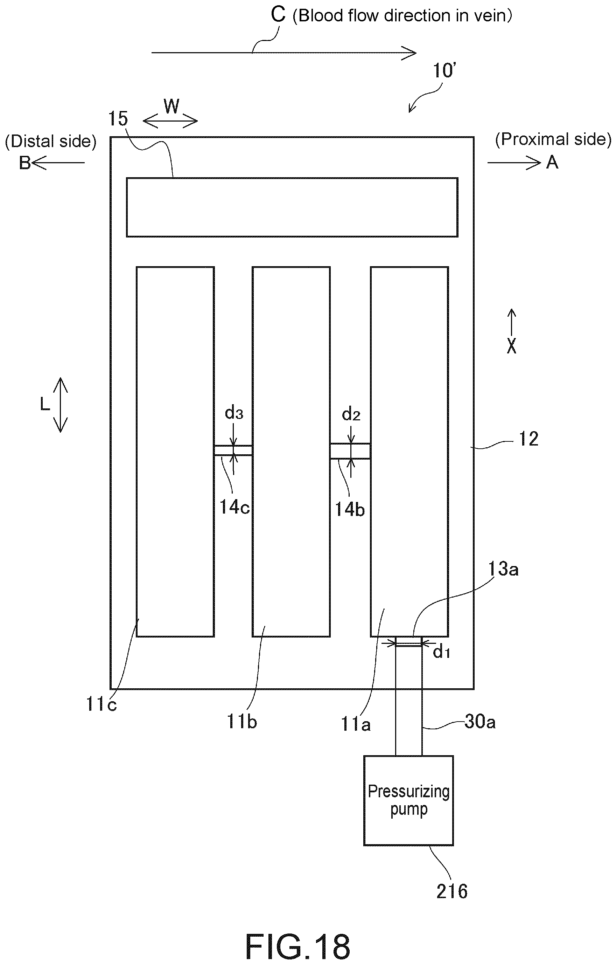

Fig. 18 is a schematic plan view showing a tourniquet according to a modified example (Part IV) of the present invention.
As shown in Fig. 18, a tourniquet 10' three pressurizing layers 11a to 11c divided in a width direction W of the tourniquet 10', for example, and a band-like cover member 12 that can be wrapped around the patient's arm which is the attached site.
The pressurizing layers 11a to 11c are disposed to be adjacent to each other in the width direction W inside the cover member 12 and the pressurizing layer 11a on the proximal side A which is one end side in the width direction is provided with a fluid introduction outlet 13a. Further, flow paths 14b, 14c for distributing pressurizing gas are provided between the adjacent pressurizing layers, i.e., between the pressurizing layer 11a and the pressurizing layer 11b and between the pressurizing layer 11b and the pressurizing layer 11c. The flow paths 14b, 14c have diameters gradually smaller from the proximal side A which is one end side in the width direction to the distal side B which is the other end side. In addition, the diameter of the fluid introduction outlet 13a provided in the pressurizing layer 14a on the proximal side A which is the one end side is larger than the diameter of the flow path 14b, 14c. That is, assuming that the diameter of the fluid introduction outlet 13a is denoted by $d_1$, the diameter of the flow path 14b is denoted by $d_2$, and the diameter of the flow path 14c is denoted by $d_3$, their relationship is as follows

$$d_1 > d_2 > d_3.$$

In this case, $d_1 : d_2 = 2 : 1$ or less is favorably set. Accordingly, it is possible to suitably delay the inflation of the pressurizing layers 11b, 11c from the inflation of the pressurizing layer 11a.
For the use, for example, the single pressurizing pump 216 supplies pressurizing gas into the pressurizing layer 11a via the air tube 30a and the fluid introduction outlet 13a. The pressurizing gas supplied into the pressurizing layer 11a is supplied into the pressurizing layer 11b via the flow path 14b. The pressurizing gas supplied into the pressurizing layer 11b is supplied into the pressurizing layer 11c via the flow path 14c.

With the tourniquet 10' having such a configuration, the pressurizing layer 11a first inflates, the pressurizing layer 11b subsequently inflates, and the pressurizing layer 11c finally inflates. That is, the region pressurized by the tourniquet 10' can be configured to expand from the proximal side A which is the one end side in the width direction W of the tourniquet 10' to the distal side B which is the other end side. In addition, the control mechanism and the like are unnecessary, and the action and effect according to the present invention can be realized with a simple configuration of the tourniquet 10', the pressurizing pump 216, and the air tube 30a according to this embodiment.

<Modified Example (Part V) of Tourniquet>

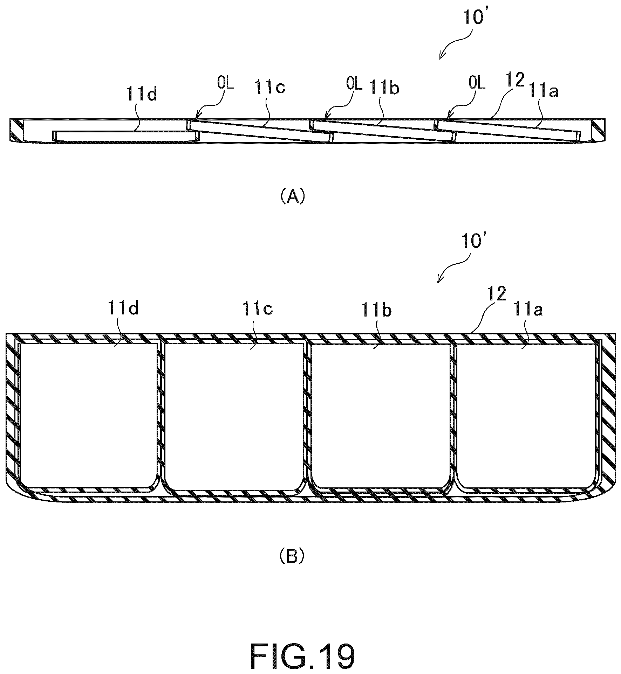

Fig. 19 is a schematic cross-sectional view showing a tourniquet according to a modified example (Part V) of the present invention. In Fig. 19, (a) shows the state of the tourniquet 10' before pressurization and (b) shows the state of the pressurized tourniquet 10'.
As shown in Fig. 19, for example, a tourniquet 10' includes four pressurizing layers 11a to 11d divided in a width direction of the tourniquet 10', for example, and a band-like cover member 12 that can be wrapped around the patient's arm which is the attached site.
As shown in (a), in the tourniquet 10' before pressurization, end portions in the width direction of the four pressurizing layers 11a to 11d having regions OL overlapping in a thickness direction of the tourniquet 10' between the adjacent pressurizing layers, i.e., between the pressurizing layer 11a and the pressurizing layer 11b, between the pressurizing layer 11b and the pressurizing layer 11c, and between the pressurizing layer 11c and the pressurizing layer 11d. The areas of the overlapping regions OL are set as appropriate to provide an action to be described later.
As shown in (b), the pressurized tourniquet 10' have no clearances between the adjacent pressurizing layers 11a to 11d, i.e., between the pressurizing layer 11a and the pressurizing layer 11b, between the pressurizing layer 11b and the pressurizing layer 11c, and between the pressurizing layer 11c and the pressurizing layer 11d.
In a case where the pressurizing layers are arranged in the tourniquet such that the overlapping regions OL are not formed between the adjacent pressurizing layers of the tourniquet before pressurization, for example, such that no clearances are formed between the adjacent pressurizing layers, there is a fear that the clearances between the adjacent pressurizing layers are formed in the pressurized tourniquet and the blood vessel at the puncture site is insufficient distended. In contrast, by providing the overlapping regions OL between the adjacent pressurizing layers, the pressurized tourniquet 10' has no clearances between the adjacent pressurizing layers. Therefore, the blood vessel at the puncture site can be reliably distended.

<Modified Example of Pressurizing Method during Additional Tourniquet Application>

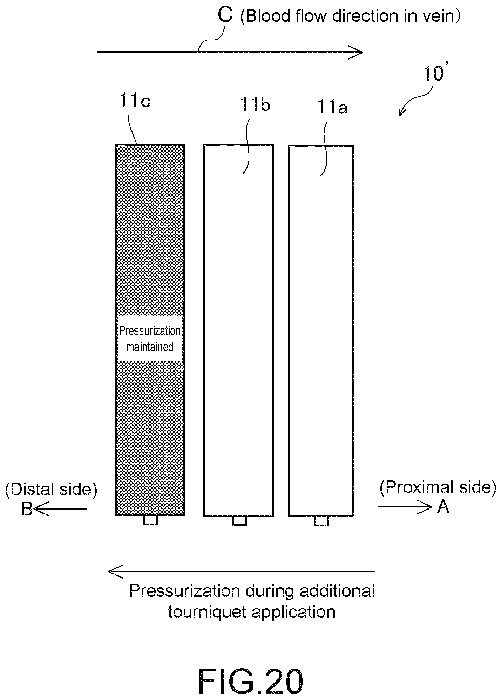

Fig. 20 is an explanatory diagram of a modified example of a pressurizing method during additional tourniquet application according to the present invention.

In Fig. 20, for the sake of simple description, an example in which the tourniquet 10' includes the three pressurizing layers 11a to 11c is shown.

In accordance with the present invention, as shown in Fig. 20, a configuration in which the pressurizing layers 11b, 11c are pressurized sequentially from the pressurizing layer 11a on the proximal side A to the patient's distal side B in the tourniquet 10' is employed. Then, during additional tourniquet application, similar pressurization is performed again. However, there has been a fear that blood flows from the patient's hand side to the heart side in veins during additional tourniquet application. In view of this, in this embodiment, during additional tourniquet application, the pressure of the pressurizing layer 11c on the distal side B is set to be higher than the suitable pressure value before the additional tourniquet application while maintaining the inflation of the pressurizing layer 11c on the distal side B. Accordingly, it is possible to suitably maintain the inflation of the pressurizing layer 11c, and the blood can be prevented from flowing from the patient's hand side to the heart side in veins during additional tourniquet application. It should be noted that the pressure set to be higher in that case may be changed as appropriate such that the blood can be prevented from flowing from the patient's hand side to the heart side in veins during additional tourniquet application.

<Others>

The present invention is not limited to the above-mentioned embodiments and various modifications and applications can be made within the scope of the technical idea of the present invention. The scope of use according to such modifications and applications also falls within the technical scope of the present invention.

For example, in any of the above-mentioned embodiments, the region pressurized by the tourniquet is configured to extend from the one end side to the other end side in the width direction of the tourniquet. However, the region pressurized by the tourniquet may be configured to move from the one end side to the other end side in the width direction of the tourniquet. For example, regarding the tourniquet 10' shown in Figs. 2 and 3 according to the above-mentioned embodiment, the pressurizing layers 11a to 11e are pressurized sequentially and all the pressurizing layers 11a to 11e are finally pressurized in the above-mentioned embodiment. However, a configuration in which the pressurizing layer 11a is first pressurized, the pressurizing layer 11b is subsequently pressurized and the pressurizing layer 11a is exhausted, and similar operations are thereafter performed up to the pressurizing layer 11e may be employed.

Further, in the hemostasis aid shown in Fig. 11, the flow paths 223 for pressure measurement are connected to the flow paths 218b between the solenoid valves 217 and the exhaust valves 218, the flow paths 223 for pressure measurement may be connected to flow paths before the solenoid valves 217 or flow paths behind the exhaust valves 218. Similarly, in the hemostasis aid 1 shown in Fig. 10, the flow paths 223 for pressure measurement are connected to the flow path 218b between the branch valve 261 and the exhaust valves 218. However, the flow paths 223 for pressure measurement may be connected to flow paths before the branch valve 261 or flow path behind the exhaust valves 218. Similarly, in the hemostasis aid 1 shown in Fig. 12, the flow paths 223 for pressure measurement are connected to the flow paths between the branching pressurizing and depressurizing pump 410 and the solenoid valves 402. However, the flow paths 223 for pressure measurement may be connected to flow paths behind the solenoid valves 402. By connecting the flow paths 223 for pressure measurement to the flow paths behind the solenoid valves 402, it is also possible to monitor leakage due to a pin-hole or the like of the pressurizing layer while the solenoid valves 402 are closed.

Further, in particular during initial blood pressure measurement, the present invention may employ heart rate control pressurizing and depressurizing method to be described below.

In the heart rate control pressurizing and depressurizing method, Korotkov sounds are measured from the start of measurement, the heart rate is measured on the basis of intervals of the sounds, and the measurement time is shortened by increasing the pressurizing and depressurizing speed when the heart rate is high. When the heart rate is low, the pressure is regulated at the depressurizing and pressurizing speed enabling the blood pressure measurement to be performed. By employing the heart rate control pressurizing and depressurizing method, the measurement can be efficiently performed for a short time for a healthy person, and it is less invasive. Further, it is theoretically expected that an effect of high measurement accuracy (during bradycardia) is provided.

The present invention may be carried out by combining the above-mentioned embodiments. For example, the embodiments shown in Figs. 16 to 20 may be combined and carried out within the scope of the technical idea of the present invention, and the scope of use also falls within the technical scope of the present invention.

Reference Signs List

1              hemostasis aid

| 10, 10' | tourniquet |
| 11a to 11e | pressurizing layer |
| 12 | cover member |
| 13, 13a to 13e | fluid introduction outlet |
| 14b, 14c | flow path |
| 20 | control device |
| 111 | pressurizing surface |
| 112 | cutout portion |
| 113 | pressurizing surface |
| 114 | pressurizing layer |
| 211 | control unit |
| 216 | pressurizing pump |
| 217 | solenoid valve |
| 219a | branch path |
| 219b | flow path |
| 224 | microphone |
| 301a to 301e | pressurizing and depressurizing pump |
| 401 | pressurizing and depressurizing pump |
| 402 | solenoid valve |
| 511 | pressurizing member |
| 512 | cover member |
| 513 | pressurizing surface |
| 514 | cutout portion |

**Claims**

1. A hemostasis aid, comprising
   a tourniquet configured such that a region pressurized by the tourniquet extends or moves from one end side to another end side in a width direction of the tourniquet.

2. The hemostasis aid according to claim 1, wherein the tourniquet includes a plurality of pressurizing layers divided in the width direction, the hemostasis aid further comprising
   a control device that performs pressurizing sequentially from a pressurizing layer on the one end side in the width direction to a pressurizing layer on the other end side.

3. The hemostasis aid according to claim 2, wherein at least one of the plurality of pressurizing layers includes a cutout portion at a corner on the other end side in a pressurizing surface of the pressurizing layer.

4. The hemostasis aid according to claim 2 or 3, wherein,
   the control device performs pressurizing sequentially from the pressurizing layer on the one end side to the pressurizing layer on the other end side again in accordance with an operation of additional tourniquet application.

5. The hemostasis aid according to claim 4, wherein the control device pressurizes, when the operation of additional tourniquet application is performed, the pressurizing layer on the other end side, which is pressurized before the re-pressurizing, until at least the pressurizing layer on the one end side is re-pressurized.

6. The hemostasis aid according to claim 4, wherein the control device pressurizes, when the operation of additional tourniquet application is performed, the pressurizing layer on the other end side, which is pressurized before the re-pressurizing, to maintain a pressure higher than a pressure before the re-pressurizing.

7. The hemostasis aid according to any one of claims 2 to 6, wherein
   the control device measures a blood pressure via the tourniquet and controls a pressure of the tourniquet to be a tourniquet pressure according to the measured blood pressure.

8. The hemostasis aid according to claim 7, wherein the control device determines a systolic pressure and a diastolic pressure on a basis of the measured blood pressure, determines a blood pressure mean value in accordance with (systolic pressure + diastolic pressure)/2, and determines a tourniquet pressure in accordance with (blood pressure mean value + diastolic pressure)/2.

9. The hemostasis aid according to any one of claims 2 to 8, wherein
   the control device includes

   a pressurizing pump that feeds a pressurizing fluid to each of the pressurizing layers,
   a branch path that branches the pressurizing fluid fed from the pressurizing pump into each of the pressurizing layers,
   a plurality of solenoid valves that opens and closes each of flow paths branched by the branch path,
   a plurality of exhaust valves for exhausting each of the flow paths branched and
   a control unit that controls an operation of the pressurizing pump, open/close of each of the solenoid valves, and open/close of each of the exhaust valves.

10. The hemostasis aid according to any one of claims 2 to 8, wherein
    the control device includes

    a pressurizing pump that feeds the pressurizing fluid into each of the pressurizing layers,
    a multi-directional solenoid valve that branches the pressurizing fluid fed from the pressurizing pump into each of the pressurizing layers and opens and closes each of flow paths branched and

a control unit that controls an operation of the pressurizing pump and open/close of the multi-directional solenoid valve.

11. The hemostasis aid according to any one of claims 2 to 8, wherein
the control device includes

a plurality of pressurizing and depressurizing pumps that is provided corresponding to the pressurizing layers, respectively, and that pressurizes and depressurizes each of the pressurizing layers and
a control unit that controls an operation of each of the pressurizing and depressurizing pumps.

12. The hemostasis aid according to any one of claims 2 to 8, wherein
the control device includes

a pressurizing and depressurizing pump that pressurizes and depressurizes each of the pressurizing layers,
a branch path that branches a pressurizing fluid fed from the pressurizing and depressurizing pump into each of the pressurizing layers,
a plurality of solenoid valves that opens and closes each of flow paths branched by the branch path and
a control unit that controls an operation of the pressurizing and depressurizing pump and open/close of each of the solenoid valves.

13. The hemostasis aid according to claim 1, wherein the tourniquet includes a pressurizing member including a pressurizing surface and a cutout portion provided at a corner on the other end side in the pressurizing surface.

14. A tourniquet, comprising:

a plurality of pressurizing layers divided in a width direction; and
a band-like cover member that is capable of being wrapped around an attached site, wherein
the plurality of pressurizing layers is disposed to be adjacent to each other in the width direction inside the cover member.

15. The tourniquet according to claim 14, wherein the pressurizing layers are respectively provided with fluid introduction outlets.

16. The tourniquet according to claim 15, wherein

the fluid introduction outlets have diameters gradually smaller sequentially from the pressurizing layer on one end side in the width direction to the pressurizing layer on another end side.

17. The tourniquet according to claim 15, wherein the pressurizing layers each have capacity gradually larger sequentially from the pressurizing layer on one end side in the width direction to the pressurizing layer on another end side.

18. The tourniquet according to claim 14, further comprising
flow paths that cause a fluid to flow between the adjacent pressurizing layers, wherein
the flow paths have diameters gradually smaller from the one end side to the other end side in the width direction, and
the pressurizing layer on the one end side includes a fluid introduction outlet having a diameter larger than the diameter of each of the flow paths.

19. A tourniquet, comprising:

a pressurizing member including

a pressurizing surface and
a cutout portion provided at a corner on one side in a width direction of the pressurizing surface; and

a band-like cover member that accommodates the pressurizing member and is capable of being wrapped around an attached site.

FIG.1

C (Blood flow direction in vein)

10

W

(Distal side)
B

15

(Proximal side)
A

X

X

L

12

13
11e

13
11d

13
11c

13
11b

13
11a

FIG.2

FIG.3

114

114

Pressurize

C (Blood flow direction in vein)

V

115 (Distal side)

B ⟵

113

(Proximal side)

116

⟶ A

113

FIG.4A

FIG.4B

FIG.5

```
                    ┌─────────────┐
                    │    Start    │
                    └─────────────┘
                           │
                           ▼
        ST401      ╱─────────────────╲
          NO     ╱    Is start         ╲
     ◄──────────  button pressed?       
               ╲                        ╱
                ╲─────────────────────╱
        ST402            │ YES
                         ▼
             ┌───────────────────────┐
             │ Measure blood pressure│
             └───────────────────────┘
                         │
                         ▼
             ┌ ─ ─ ─ ─ ─ ─ ─ ─ ─ ─ ┐
               Measure pulse waves
             │ and generate blood   │
               pressure waveform
             └ ─ ─ ─ ─ ─ ─ ─ ─ ─ ─ ┘
                         │        ST403
                         ▼
               ╱─────────────╲
              ╱  Can blood     ╲     NO                    ST406
             ╱  pressure be     ╲──────────────┐
             ╲   measured?      ╱               ▼
              ╲               ╱        ┌──────────────────┐
               ╲─────────────╱         │ Output measurement│
                    │ YES    ST404     │   error alarm     │
                    ▼                  └──────────────────┘
             ┌───────────────────────┐          │
             │ Maintain pressure such│          ▼
             │ that pressure can be  │    ┌──────────┐
             │ maintained at middle  │    │   End    │
             │ point between mean    │    └──────────┘
             │ blood pressure and    │
             │ diastolic pressure    │
             └───────────────────────┘
                         │  ST405
                         ▼
               ╱─────────────╲
              ╱  Has certain   ╲   YES                    ST408
             ╱  time elapsed?   ╲─────────────┐
             ╲                  ╱              ▼
              ╲───────────────╱       ┌──────────────────┐
                    │ NO   ST407      │ Output notification│
                    ▼                 │     buzzer        │
               ╱─────────────╲        └──────────────────┘
              ╱     Is         ╲   YES           │
             ╱ additional       ╲────────────────┘
             ╲ tourniquet       ╱
              ╲ application     ╱
               ╲ button        ╱
                ╲ pressed?    ╱
                 ╲──────────╱
              NO     │    ST409
              ST411  ▼                          ST410
               ╱─────────────╲
          NO  ╱     Is end     ╲
     ◄────────  button pressed? 
             ╲                ╱         ┌──────────────────┐
              ╲─────────────╱           │ Perform additional│
                    │ YES               │tourniquet applicat.│
                    ▼                   └──────────────────┘
             ┌───────────────────┐
             │   Depressurize    │ ST412
             │ tourniquet quickly│
             └───────────────────┘
                    │
                    ▼
             ┌──────────┐
             │   End    │
             └──────────┘
```

FIG.6

FIG.7

FIG.8

FIG.9

FIG.10

FIG.11

1

20

215

Input operation unit

402

219b

Solenoid valve

Solenoid valve

219a

Solenoid valve

Solenoid valve

Pressurizing and depressurizing pump

Solenoid valve

10

Tourniquet

224

Storage unit

212

213

Display unit

21

Control unit

214

Notification unit

211

401

Measurement unit

222

Pressure gauge

221

223

22

FIG.12

End button  On / Off ⟋(8)

Additional tourniquet application button  On / Off

Start button  On / Off ⟋(1)

Pressuring layer a  Tourniquet pressure / O  (2) (3)

Pressuring layer b  Tourniquet pressure / O  (4)

Pressuring layer c  Tourniquet pressure / O  (5)

Pressuring layer d  Tourniquet pressure / O  (2') (6)

Pressuring layer e  Tourniquet pressure / O  (7)

↑Pressure
Time→

Blood pressure measurement | Tourniquet application operation→ | (9)

## FIG.13

FIG.14

10'

W

C (Blood flow direction in vein)

511   512

514   513

(Distal side)
B

(Proximal side)
A

FIG.15

FIG.16

C (Blood flow direction in vein)

10'

W

15

(Distal side)
B←

(Proximal side)
→A

L

X

12

Q₃    Q₂    Q₁

11c 13c    11b 13b    11a 13a

# FIG.17

FIG.18

10'

11d    0L  11c    0L  11b    0L  12 11a

(A)

10'

11d        11c        11b      12 11a

(B)

FIG.19

C (Blood flow direction in vein)

11c          11b          11a

10'

Pressurization
maintained

(Distal side)                                    (Proximal side)
B ←——                                            ——→ A

←————————————————————————
Pressurization during additional
tourniquet application

FIG.20

## INTERNATIONAL SEARCH REPORT

| | International application No. |
|---|---|
| | PCT/JP2019/018813 |

**A. CLASSIFICATION OF SUBJECT MATTER**
Int.Cl. A61B5/022(2006.01)i, A61B5/0225(2006.01)i, A61B5/153(2006.01)n, A61B17/132(2006.01)n

According to International Patent Classification (IPC) or to both national classification and IPC

**B. FIELDS SEARCHED**

Minimum documentation searched (classification system followed by classification symbols)
Int.Cl. A61B5/022, A61B5/0225, A61B5/153, A61B17/132

Documentation searched other than minimum documentation to the extent that such documents are included in the fields searched
Published examined utility model applications of Japan    1922–1996
Published unexamined utility model applications of Japan    1971–2019
Registered utility model specifications of Japan    1996–2019
Published registered utility model applications of Japan    1994–2019

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| X<br>Y<br>A | JP 53-126787 A (WHITNEY, J. K.) 06 November 1978, page 3, upper left column, line 10 to page 4, lower left column, line 2, fig. 1-10<br>& US 4453538 A, column 2, line 34 to column 4, line 9, fig. 1-10 & DE 2814691 A1 | 1-3, 13-15, 19<br>4-7, 9-12<br>8, 16-18 |
| X<br>Y<br>A | US 5968073 A (JACOBS, L. F.) 19 October 1999, column 5, line 9 to column 7, line 33, fig. 2, 3<br>& EP 1060729 A1 | 1-2, 14-15, 17<br>3-7, 9-13, 19<br>8, 16, 18 |

☒ Further documents are listed in the continuation of Box C.    ☐ See patent family annex.

| | | |
|---|---|---|
| * Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family | |

| Date of the actual completion of the international search | Date of mailing of the international search report |
|---|---|
| 07.06.2019 | 18.06.2019 |

| Name and mailing address of the ISA/<br>Japan Patent Office<br>3-4-3, Kasumigaseki, Chiyoda-ku,<br>Tokyo 100-8915, Japan | Authorized officer |
|---|---|
| | Telephone No. |

Form PCT/ISA/210 (second sheet) (January 2015)

**EP 3 791 781 A1**

**INTERNATIONAL SEARCH REPORT**

International application No.

PCT/JP2019/018813

C (Continuation). DOCUMENTS CONSIDERED TO BE RELEVANT

| Category* | Citation of document, with indication, where appropriate, of the relevant passages | Relevant to claim No. |
|---|---|---|
| X<br>Y | JP 2009-297223 A (OMRON HEALTHCARE CO., LTD.) 24 December 2009, paragraphs [0024]-[0024], [0031], [0040]-[0045], [0049]-[0051], fig. 1-3, 10 (Family: none) | 1-2, 4-7, 14-15<br>3, 9-13, 19 |
| X | WO 2016/163326 A1 (TERUMO CORPORATION) 13 October 2016, paragraph [0075], fig. 1, 14 & US 2018/0042615 A1, paragraph [0089], fig. 1, 14 | 1-3, 13-15, 17, 19 |
| Y | JP 7-51276 A (ULRICH, H.) 28 February 1995, paragraphs [0010]-[0014], fig. 1 & US 5569304 A, column 3, line 41 to column 5, line 62, fig. 1 & DE 4317600 A1 | 4-7 |
| Y | JP 58-501616 A (VARIX, HB) 29 September 1983, specification, page 2, lower right column, line 3 to page 3, upper left column, line 7, fig. 2c & US 4566436 A, column 2, line 61 to column 4, line 5, fig. 2c | 3, 13, 19 |
| Y | JP 2014-204969 A (WATANABE, Koo) 30 October 2014, paragraphs [0041]-[0048], fig. 7 (Family: none) | 9-12 |
| A | JP 2003-290156 A (MATSUSHITA ELECTRIC WORKS, LTD.) 14 October 2003, fig. 5, 6 (Family: none) | 18 |

Form PCT/ISA/210 (continuation of second sheet) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2013118938 A

- JP 2017113581 A